# FASCICULE DE BREVET EUROPEEN

(11) **EP 1 105 102 B1**
(45) Date de publication et mention de la délivrance du brevet: **02.01.2003**
(21) Numéro de dépôt: 99936748.5
(22) Date de dépôt: 18.08.1999
(51) Int. Cl.: A61K 9/19, A61K 31/4365

(54) **COMPOSITION PHARMACEUTIQUE INJECTABLE A BASE D'UN SEL PHARMACEUTIQUEMENT ACCEPTABLE DU CLOPIDOGREL OU DE TICLOPIDINE**
INJIZIERBARES ARZNEIMITTEL AUF BASIS EINES PHARMAZEUTISCH VERTRÄGLICHEN SALZES VON CLOPIDOGREL BZW. VON TICLOPIDIN
PHARMACEUTICAL COMPOSITION FOR INJECTION BASED ON A PHARMACEUTICALLY ACCEPTABLE CLOPIDOGREL OR TICLOPIDIN SALT

(30) Priorité: 20.08.1998 FR 9810567
(43) Date de publication de la demande: 13.06.2001
(73) Titulaire: SANOFI-SYNTHELABO, 75013 Paris (FR)
(72) Inventeur: ALEMAN, Claude Le Logis des Pins, F-34270 Valflaunes (FR); BREUL, Thierry, F-34110 Frontignan (FR)
(74) Mandataire: Thouret-Lemaitre, Elisabeth
(86) Numéro de dépôt international: FR9902003
(87) Numéro de publication internationale: WO00010534

(56) Documents cités:
- EP-A- 0 705 601
- EP-A- 0 742 013
- WO-A-97/17064

## Description

La présente invention concerne une nouvelle composition pharmaceutique injectable à base d'un sel pharmaceutiquement acceptable du clopidogrel ou de la ticlopidine.

En particulier, la présente invention a pour objet une formulation injectable de dopidogrel sous forme hydrogénosulfate.

Le clopidogrel est un nouvel antiagrégant plaquettaire, notamment décrit dans les brevets EP 099802, EP 281459 et US 4,847,265. Ce médicament est plus particulièrement utilisé pour la réduction des accidents liés à l'athérosclérose (infarctus du myocarde, accident vasculaire cérébral, artérite, mort vasculaire) chez les patients ayant eu un infarctus du myocarde récent, un accident vasculaire cérébral récent ou présentant une artériopathie oblitérante des membres inférieurs avérée. La ticlopidine est également un puissant agent antithrombotique ; on pourra se référer à FR 73 03503. Le clopidogrel et la ticlopidine doivent souvent être administrés dans l'urgence à des patients pour lesquels l'administration par voie orale est difficile.

Le clopidogrel est commercialisé sous sa forme hydrogénosulfate et la ticlopidine sous sa forme chlorhydrate. Aucune formulation injectable à base du clopidogrel sous forme hydrogénosulfate ou à base de la ticlopidine sous forme chlorhydrate n'est commercialisée.

Dans les brevets US 4,847,265, EP 099 802 et EP 281459 une composition à base de clopidogrel ou d'un de ses sels pharmaceutiquement acceptables pour administration parentérale, sous forme de soluté injectable est mentionnée. Ce type de formulation contenant un sel du clopidogrel en solution dans un solvant isotonique est difficilement utilisable. En effet, ce type de solution dont le pH est inférieur à 2 rend l'injection très douloureuse.

Dans la demande de brevet WO 97/17064, on décrit une forme lyophilisée du clopidogrel ou d'un de ses sels pharmaceutiquement acceptable contenant du mannitol et de l'alanine. Lors de la reconstitution du lyophilisat dans le solvant de reprise, on observe souvent un problème d'auto-agrégation du clopidogrel.

Ainsi, la préparation d'une forme injectable à base d'un sel pharmaceutiquement acceptable du clopidogrel ou de la ticlopidine est difficile à cause des propriétés physico-chimiques de ces principes actifs.

Il a maintenant été trouvé une nouvelle forme injectable d'un sel pharmaceutiquement acceptable du clopidogrel ou de la ticlopidine, beaucoup mieux tolérée à l'injection et plus facilement manipulable pour la préparation de la forme galénique.

Par forme injectable, on entend un lyophilisat reconstitué dans un solvant de reprise. Les sels du clopidogrel et de la ticlopidine sont instables en solution aqueuse : on observe une forte dégradation. Il est donc nécessaire de réaliser un lyophilisat stable à température ambiante qui sera reconstitué dans un solvant de reprise avant l'injection.

Les sels du clopidogrel ou de la ticlopidine, lyophilisés seuls, forment des agrégats insolubles collés au verre du flacon. Il s'est donc révélé indispensable d'utiliser un inhibiteur d'agrégation. Le poloxamer 188, copolymère d'oxyde d'éthylène et d'oxyde de propylène (masse moléculaire = 8350) commercialisé sous le nom de Pluronic F68® s'est révélé être un tensioactif injectable particulièrement efficace pour l'obtention d'un lyophilisat à la fois non effondré dont le temps de dissolution dans le solvant de reprise est inférieur à une minute et qui est stable physiquement au moins pendant une semaine à une température comprise entre 5°C et 40°C.

Ainsi, la présente invention concerne une formulation lyophilisée contenant en tant que principe actif un sel pharmaceutiquement acceptable du clopidogrel ou de la ticlopidine et du Pluronic F68®.

La formulation lyophilisée selon l'invention comprend avantageusement 65 à 95 % (p/p) du sel pharmaceutiquement acceptable du clopidogrel ou de la tictopidine et 5 à 35 % (p/p) de Pluronic F68®.

Plus particulièrement, la présente invention a pour objet une formulation lyophilisée qui comprend 65 à 95 % (p/p) d'un sel pharmaceutiquement acceptable du clopidogrel et 5 à 35 % (p/p) de Pluronic F68®

Particulièrement, l'invention concerne une formulation lyophilisée contenant le clopidogrel sous forme hydrogénosulfate et du Pluronic F68®.

Selon la présente invention, une formulation lyophilisée contenant 80 % (p/p) de clopidogrel sous forme hydrogénosulfate et 20 % (p/p) de Pluronic F68® est particulièrement préférée.

Pour obtenir une composition aqueuse injectable, ce lyophilisât doit être mis en solution.

Comme indiqué précédemment, les sels du clopidogrel et de la ticlopidine sont des acides forts. En solution aqueuse, ils donnent des solutions présentant un pH inférieur à 2, ce qui est incompatible avec l'injection.

Il est donc nécessaire d'utiliser un agent basique modificateur de pH de préférence le phosphate disodique pour obtenir une solution tamponnée de pH supérieur à 4. A un tel pH, les sels du clopidogrel et de la ticlopidine précipitent ce qui constitue un inconvénient majeur pour une forme injectable.

Il a maintenant été trouvé que, pour avoir une solution compatible avec l'injection, le lyophilisât doit être reconstitué dans un solvant de reprise contenant à la fois un agent modificateur de pH compatible avec l'injection et plus particulièrement le phosphate disodique pour obtenir un pH compris entre 4 et 7 et du polyoxyéthylèneglycol-660-12- hydroxystéarate de formule : 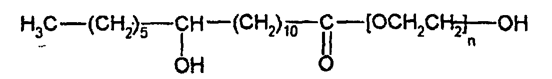 commercialisé sous le nom de Solutol HS15® pour éviter la précipitation du principe actif à ce pH. Le Solutol HS15® est un retardateur de précipitation particulièrement efficace qui permet d'obtenir une solution compatible avec l'injection.

Le solvant de reprise contient un agent basique modificateur de pH de préférence le phosphate disodique; de telles bases sont utilisées en quantité proche de la stoechiométrie par rapport au principe actif utilisé.

Le Solutol HS15® est ajouté au solvant de reprise à raison de 5 à 15 % (p/v) de préférence à raison d'environ 9 % (p/v).

Ainsi, la présente invention a donc pour objet une composition aqueuse injectable contenant un sel pharmaceutiquement acceptable du clopidogrel ou de la ticlopidine, du Pluronic F68®, un agent basique modificateur de pH compatible avec l'administration par injection et du Solutol HS15®.

Selon la présente invention, l'eau utilisée est convenable pour les préparations injectables.

De manière préférée, la composition injectable selon l'invention comprend une quantité de sel pharmaceutiquement acceptable du clopidogrel ou de la ticlopidine, comprise entre 0,1 et 20 % (p/v), de préférence entre 0,5 et 4 % (p/v).

En particulier, la composition injectable selon l'invention contient le clopidogrel sous forme hydrogénosulfate, de préférence 2 % (p/v) de clopidogrel sous forme hydrogénosulfate.

Une composition injectable selon l'invention particulièrement préférée contient 2 % (p/v) de clopidogrel sous forme hydrogénosulfate, le phosphate disodique, de préférence en quantité proche de la stoechiométrie par rapport au clopidogrel, du Pluronic F68® et du Solutol HS15®.

Encore plus préférées sont les compositions injectables selon l'invention contenant 2 % (p/v) de clopidogrel sous forme hydrogénosulfate, le phosphate disodique, de préférence en quantité proche de la stoechiométrie par rapport au clopidogrel, 0,01 à 2 % (p/v) de Pluronic F68®, de préférence 0,5 % (p/v) et du Solutol HS15®.

De manière particulièrement préférée , l'invention concerne des compositions injectables contenant 2 % (p/v) de clopidogrel sous forme hydrogénosulfate, le phosphate disodique, de préférence en quantité proche de la stoechiométrie par rapport au clopidogrel, 0,5 % (p/v) de Pluronic F68® et 5 à 15 % (p/v) de Solutol HS15® de préférence 9 % (p/v).

En particulier, la présente invention a pour objet une composition injectable constituée d'un lyophilisat qui comprend 80 % (p/p) de clopidogrel sous forme hydrogénosulfate, et 20 % (p/p) de Pluronic F68®, le dit lyophilisat étant reconstitué dans un solvant de reprise, solution aqueuse qui comprend en quantité stoechiométrique par rapport au principe actif un agent basique modificateur de pH, de préférence le phosphate disodique et 9 % (p/v) de Solutol HS15®.

Les lyophilisats de la présente invention sont préparés par des procédés classiques, selon les techniques habituelles de lyophilisation. Ainsi, par exemple, on dissout le principe actif et le Pluronic F68® sous agitation dans une quantité appropriée d'eau pour préparation injectable. La solution obtenue est clarifiée et filtrée dans des conditions stériles puis introduite dans des récipients (flacons) stériles à la capacité voulue.

La lyophilisation des solutions est réalisée comme suit : la solution suit un cycle de congélation puis de sublimation et de dessiccation adapté au volume à lyophiliser et au récipient contenant la solution. Par exemple, on procède à la congélation de la solution à une température comprise entre - 15 et - 80°C de préférence entre - 40 et - 50°C, pendant une durée comprise entre 2 et 5 heures puis à la sublimation, à une température comprise entre - 10 et - 50°C, de préférence entre - 30 et - 40°C sous une pression réduite comprise entre 3 et 60 Pa de préférence entre 10 et 14 Pa pendant un temps compris entre 60 et 100 heures et enfin à la dessication secondaire à une température comprise entre 0 et 35°C de préférence entre 20 et 30°C sous une pression comprise entre 0,5 et 20 Pa, de préférence entre 4 et 5 Pa, pendant un temps compris entre 10 et 40 heures. On bouche ensuite les flacons dans des conditions stériles habituelles.

Le solvant de reprise est préparé par dissolution sous agitation dans une quantité appropriée d'eau pour préparation injectable de Solutol HS15® préalablement fondu et de l'agent basique. La solution est alors convenablement filtrée, pour éliminer des résidus éventuels, et stérilisée. De préférence, avant la stérilisation effectuée dans un autoclave, la solution est subdivisée dans des ampoules ou flacons mono-dose, éventuellement en opérant sous atmosphère d'azote.

La préparation injectable peut être prête à l'emploi ou être reconstituée juste avant l'injection. Dans le cas où la composition injectable est reconstituée juste avant l'injection, la préparation pharmaceutique pourra se présenter sous la forme d'une poudre lyophilisée, contenue dans un flacon, associée à un solvant de reconstitution contenu dans un autre flacon.

La préparation pharmaceutique pourra également se présenter sous la forme d'une poudre lyophilisée associée à un solvant de reconstitution, les deux formes étant contenues dans une seringue à double compartiment.

Ainsi, selon un autre de ses aspects, la présente invention a pour objet un kit pharmaceutique qui se présente sous la forme de deux récipients stériles, l'un renfermant une formulation lyophilisée qui contient en tant que principe actif un sel pharmaceutiquement acceptable du clopidogrel ou de la ticlopidine et du Pluronic F68® et l'autre renfermant un solvant de reprise aqueux qui comprend un agent basique modificateur de pH compatible avec l'administration par injection et du Solutol HS15®.

La présente invention a également pour objet un kit pharmaceutique qui se présente sous la forme d'une seringue double compartiment, l'un des compartiments renfermant une formulation lyophilisée qui contient en tant que principe actif un sel pharmaceutiquement acceptable du clopidogrel ou de la ticlopidine et du Pluronic F68® et l'autre compartiment renfermant un solvant de reprise aqueux qui comprend un agent basique modificateur de pH compatible avec l'administration par injection et du Solutol HS15®.

En particulier, les kits pharmaceutiques selon la présente invention comprennent la formulation lyophilisée contenant en tant que principe actif le clopidogrel sous forme hydrogénosulfate ; de préférence la formulation lyophilisée contient 80 % (p/p) de clopidogrel sous forme hydrogénosulfate et 20 % (p/p) de Pluronic F68®.

Plus particulièrement, la présente invention concerne des kits pharmaceutiques dans lesquels la formulation lyophilisée contient 80 % (p/p) de clopidogrel sous forme hydrogénosulfate et 20 % (p/p) de Pluronic F68® et le solvant de reprise comprend le phosphate disodique en tant qu'agent basique, de préférence, en quantité proche de la stoechiométrie par rapport au principe actif et du Solutol HS15®.

Particulièrement préférés sont les kits pharmaceutiques dans lesquels la formulation lyophilisée contient 80 % (p/p) de clopidogrel sous forme hydrogénosulfate et 20 % (p/p) de Pluronic F68® et le solvant de reprise comprend le phosphate disodique en tant qu'agent basique, en quantité proche de la stoechiométrie par rapport au principe actif et 5 à 15 % (p/v) de Solutol HS15®, de préférence 9% (p/v).

Selon un autre de ces aspects, la présente invention a également pour objet l'utilisation du clopidogrel ou de la ticlopidine ou un de leurs sels pharmaceutiquement acceptables pour la préparation de formulations injectables convenables pour la réduction des accidents liés à l'athérosclérose chez des patients pour lesquels l'administration de médicaments par voie orale est impossible.

Plus particulièrement, la présente invention a pour objet l'utilisation du clopidogrel sous forme hydrogénosulfate pour la préparation de formulations injectables convenables pour la réduction des accidents liés à l'athérosclérose chez des patients pour lesquels l'administration de médicaments par voie orale est impossible. La formulation injectable selon l'invention sera administrée en dose unique ou pendant un à cinq jours dans les états aigus pour initier le traitement.

La dose journalière de principe actif à administrer varie naturellement selon l'âge et le poids du patient, ainsi que selon le type et la gravité de la pathologie à traiter.

En général, pour un adulte ayant une constitution normale, la dose journalière de principe actif à administrer selon l'invention est comprise entre 1 et 500 mg, de préférence entre 25 et 125 mg.

Les doses unitaires peuvent donc contenir de 25 à 125 mg de principe actif. De telles doses unitaires, après une convenable dilution dans la solution à injecter, sont en général administrées une fois par jour.

Selon un aspect préféré, les doses unitaires contiennent 75 mg de clopidogrel dans un volume de 5 ml. Selon un aspect encore plus préféré, les doses unitaires contiennent 100 mg de clopidogrel sous forme hydrogénosulfate dans un volume de 5 ml.

L'EXEMPLE qui suit illustre l'invention sans toutefois la limiter.

### EXEMPLE 1 : Clopidogrel sous forme hydrogénosulfate

**TABLEAU I**

| Composition de la solution à lyophiliser : | |
|---|---|
| **Constituant** | **Quantité** |
| Clopidogrel sous forme hydrogénosulfate | 100 mg * |
| Pluronic F68® | 25 mg |
| Eau pour préparation injectable | 5 ml |

| | |
|---|---|
| * correspondant à 75 mg de clopidogrel. | |

Le clopidogrel sous forme hydrogénosulfate et le Pluronic F68® sont ajoutés dans l'eau pour préparation injectable à température ambiante sous agitation légère jusqu'à dissolution complète pendant au moins 30 minutes. La solution ainsi obtenue est filtrée sur un filtre de seuil de rétention de 0,22 µm et répartie en flacons de verre à raison de 5 ml par flacon. Les flacons sont bouchés par des bouchons piliers pour lyophilisation en chlorobutyle. Les flacons ainsi obtenus sont lyophilisés selon un cycle de lyophilisation approprié, par exemple :
- Congélation à - 45°C pendant 3 heures
- Sublimation à - 35°C sous une pression de 12 Pa pendant 80 heures
- Dessiccation secondaire à 25°C sous une pression de 3 Pa pendant 20 heures.

Les flacons obtenus sont alors bouchés sous courant d'azote puis sertis par des capsules d'aluminium.

**TABLEAU II**

| Composition du solvant de reprise du lyophilisat | |
|---|---|
| **Constituant** | **Quantité** |
| Solutol HS 15® | 450 mg |
| Na₂HPO₄, 12 H₂O | 179 mg |
| Eau pour préparation injectable | 5 ml |

Le Solutol HS 15® est préalablement chauffé à 50°C afin d'être à l'état fondu.

Le Solutol HS 15® fondu et le phosphate disodique sont alors ajoutés dans l'eau pour préparation injectable à température ambiante sous agitation légère jusqu'à dissolution complète pendant au moins 30 minutes.

La solution ainsi obtenue est filtrée sur un filtre de seuil de rétention de 0,22 µm et répartie en flacons de verre à raison de 5 ml par flacon. Les flacons sont ensuite bouchés avec des bouchons en chlorobutyle puis sertis par des capsules d'aluminium puis autoclavés à 121°C pendant un temps compris entre 15 min et 30 min pour garantir leur stérilité.

Les flacons sont ensuite agités afin d'éviter un éventuel déphasage de la solution obtenue lors de l'autoclavage.

## Revendications

1. Composition aqueuse injectable **caractérisée en ce qu'**elle contient en tant que principe actif un sel pharmaceutiquement acceptable du clopidogrel ou de la ticlopidine, du poloxamer 188, copolymère d'oxyde d'éthylène et d'oxyde de propylène (masse moléculaire = 8350) commercialisé sous le nom de Pluronic F68®, un agent basique modificateur de pH compatible avec l'administration par injection et du polyoxyéthylèneglycol-660-12-hydroxystéarate commercialisé sous le nom de Solutol HS15®.

2. Composition aqueuse injectable selon la revendication 1 **caractérisée en ce que** le principe actif est le clopidogrel sous forme hydrogénosulfate.

3. Composition aqueuse injectable selon la revendication 2 **caractérisée en ce qu'**elle contient une quantité de clopidogrel sous forme hydrogénosulfate comprise entre 0,1 et 20 % (p/v).

4. Composition aqueuse injectable selon la revendication 3 **caractérisée en ce qu'**elle contient une quantité de clopidogrel sous forme hydrogénosulfate comprise entre 0,5 et 4 % (p/v).

5. Composition aqueuse injectable selon la revendication 4 **caractérisée en ce qu'**elle contient une quantité de clopidogrel sous forme hydrogénosulfate égale à 2 % (p/v).

6. Composition aqueuse injectable selon l'une quelconque des revendications 1 à 5 **caractérisée en ce que** l'agent basique est le phosphate disodique.

7. Composition aqueuse injectable selon la revendication 6 **caractérisée en ce que** le phosphate disodique est présent en quantité proche de la stoechiométrie par rapport au principe actif.

8. Composition aqueuse injectable selon la revendication 7 **caractérisée en ce qu'**elle comprend de 0,01 à 2 % (p/v) de Pluronic F68®.

9. Composition aqueuse injectable selon la revendication 8 **caractérisée en ce qu'**elle comprend 0,5 % (p/v) de Pluronic F68®.

10. Composition aqueuse injectable selon la revendication 9 **caractérisée en ce qu'**elle comprend de 5 à 15 % (p/v) de Solutol HS15®.

11. Composition selon la revendication 10 **caractérisée en ce qu'**elle comprend 9 % (p/v) de Solutol HS15®.

12. Composition selon l'une quelconque des revendications 1 à 11 **caractérisée en ce qu'**elle est constituée d'une formulation lyophilisée qui comprend 80 % (p/p) de clopidogrel sous forme hydrogénosulfate et 20 % (p/p) de Pluronic F68®, le dit lyophilisât étant reconstitué dans un solvant de reprise, solution aqueuse qui comprend en quantité stoechiométrique par rapport au principe actif le phosphate disodique et 9 % (p/v) de Solutol HS15®.

13. Formulation lyophilisée **caractérisée en ce qu'**elle contient en tant que principe actif un sel pharmaceutiquement acceptable du clopidogrel ou de la ticlopidine et du Pluronic F68®.

14. Formulation lyophilisée selon la revendication 13 **caractérisée en ce que** le principe actif est le clopidogrel sous forme hydrogénosulfate.

15. Formulation lyophilisée selon la revendication 14 **caractérisée en ce qu'**elle contient 80 % (p/p) de clopidogrel sous forme hydrogénosulfate et 20 % (p/p) de Pluronic F68®.

16. Kit pharmaceutique **caractérisé en ce qu'**il se présente sous la forme de deux récipients stériles, l'un renfermant une formulation lyophilisée selon l'une quelconque des revendications 13 à 15 et l'autre renfermant un solvant de reprise aqueux qui comprend un agent basique modificateur de pH compatible avec l'administration par injection et du Solutol HS15®.

17. Kit pharmaceutique **caractérisé en ce qu'**il se présente sous la forme d'une seringue double compartiment, l'un des compartiments renfermant une formulation lyophilisée selon l'une quelconque des revendications 13 à 15 et l'autre compartiment renfermant un solvant de reprise aqueux qui comprend un agent basique modificateur de pH compatible avec l'administration par injection et du Solutol HS15®.

18. Kit pharmaceutique selon l'une quelconque des revendications 16 à 17 **caractérisé en ce que** le solvant de reprise comprend le phosphate disodique en tant qu'agent basique.

19. Kit pharmaceutique selon la revendication 18 **caractérisé en ce que** le solvant de reprise contient le phosphate disodique en quantité proche de la stoechiométrie par rapport au principe actif.

20. Kit pharmaceutique selon la revendication 19 **caractérisé en ce que** le solvant de reprise comprend de 5 à 15 % (p/v) de Solutol HS15®.

21. Kit pharmaceutique selon la revendication 20 **caractérisé en ce que** le solvant de reprise comprend 9% (p/v) de Solutol HS15®.

## Claims

1. Aqueous composition for injection, **characterized in that** it contains, as active ingredient, a pharmaceutically acceptable salt of clopidogrel or of ticlopidine, of poloxamer 188, a copolymer of ethylene oxide and propylene oxide (molecular mass = 8350) marketed under the name Pluronic F68®, a basic pH-modifying agent compatible with administration by injection and polyoxyethylene glycol 660 12-hydroxystearate marketed under the name Solutol HS15®.

2. Aqueous composition for injection according to Claim 1, **characterized in that** the active ingredient is clopidogrel in hydrogen sulphate form.

3. Aqueous composition for injection according to Claim 2, **characterized in that** it contains a quantity of clopidogrel in hydrogen sulphate form of between 0.1 and 20% (w/v).

4. Aqueous composition for injection according to Claim 3, **characterized in that** it contains a quantity of clopidogrel in hydrogen sulphate form of between 0.5 and 4% (w/v).

5. Aqueous composition for injection according to Claim 4, **characterized in that** it contains a quantity of clopidogrel in hydrogen sulphate form equal to 2% (w/v).

6. Aqueous composition for injection according to any one of Claims 1 to 5, **characterized in that** the basic agent is disodium phosphate.

7. Aqueous composition for injection according to Claim 6, **characterized in that** the disodium phosphate is present in a quantity close to stoichiometry relative to the active ingredient.

8. Aqueous composition for injection according to Claim 7, **characterized in that** it comprises from 0.01 to 2% (w/v) of Pluronic F68®.

9. Aqueous composition for injection according to Claim 8, **characterized in that** it comprises 0.5% (w/v) of Pluronic F68®.

10. Aqueous composition for injection according to Claim 9, **characterized in that** it comprises from 5 to 15% (w/v) of Solutol HS15®.

11. Composition according to Claim 10, **characterized in that** it comprises 9% (w/v) of Solutol HS15®.

12. Composition according to any one of Claims 1 to 11, **characterized in that** it constitutes a freeze-dried formulation which comprises 80% (w/w) of clopidogrel in hydrogen sulphate form and 20% (w/w) of Pluronic F68®, the said freeze-dried product being reconstituted in a reconstitution solvent, an aqueous solution which comprises, in a stoichiometric quantity relative to the active ingredient, disodium phosphate and 9% (w/v) of Solutol HS15®.

13. Freeze-dried formulation, **characterized in that** it contains, as active ingredient, a pharmaceutically acceptable salt of clopidogrel or ticlopidine and Pluronic F68®.

14. Freeze-dried formulation according to Claim 13, **characterized in that** the active ingredient is clopidogrel in hydrogen sulphate form.

15. Freeze-dried formulation according to Claim 14, **characterized in that** it contains 80% (w/w) of clopidogrel in hydrogen sulphate form and 20% (w/w) of Pluronic F68®.

16. Pharmaceutical kit, **characterized in that** it is provided in the form of two sterile containers, one containing a freeze-dried formulation according to any one of Claims 13 to 15 and the other containing an aqueous reconstitution solvent which comprises a basic pH-modifying agent compatible with administration by injection and Solutol HS15®.

17. Pharmaceutical kit, **characterized in that** it is provided in the form of a double-compartment syringe, one of the compartments containing a freeze-dried formulation according to any one of Claims 13 to 15 and the other compartment containing an aqueous reconstitution solvent which comprises a basic pH-modifying agent compatible with administration by injection and Solutol HS15®.

18. Pharmaceutical kit according to either of Claims 16 and 17, **characterized in that** the reconstitution solvent comprises disodium phosphate as basic agent.

19. Pharmaceutical kit according to Claim 18, **characterized in that** the reconstitution solvent contains disodium phosphate in a quantity close to stoichiometry relative to the active ingredient.

20. Pharmaceutical kit according to Claim 19, **characterized in that** the reconstitution solvent comprises from 5 to 15% (w/v) of Solutol HS15®.

21. Pharmaceutical kit according to Claim 20, **characterized in that** the reconstitution solvent comprises 9% (w/v) of Solutol HS15®.

## Patentansprüche

1. Wäßrige, injizierbare Zubereitung, **dadurch gekennzeichnet, daß** sie als Wirkstoff ein pharmazeutisch annehmbares Salz von Clopidogrel oder von Ticiopidin. Poloxamer 188, das unter der Bezeichnung Pluroinic F68® vertriebene Ethylenoxid /Propylenoxid-Copolymer (Molekülmasse = 8350), ein mit der Verabreichung durch Injektion verträgliches basisches Mittel zur Modifizierung des pH-Werts und das unter der Bezeichnung Solutol HS15® im Handel vertriebene Polyoxyethylenglykol-660-12-hydroxystearat enthält.

2. Wäßrige, injizierbare Zubereitung nach Anspruch 1, **dadurch gekennzeichnet, daß** der Wirkstoff Clopidogrel in Form des Hydrogensulfats ist.

3. Wäßrige, injizierbare Zubereitung nach Anspruch 2, **dadurch gekennzeichnet, daß** sie Clopidogrel in Form des Hydrogensulfats in einer Menge zwischen 0,1 und 20 % (Gew./Vol.) enthält.

4. Wäßrige, injizierbare Zubereitung nach Anspruch 3, **dadurch gekennzeichnet, daß** sie Clopidogrel in Form des Hydrogensulfats in einer Menge zwischen 0,5 und 4 % (Gew./Vol.) enthält.

5. Wäßrige, injizierbare Zubereitung nach Anspruch 4, **dadurch gekennzeichnet, daß** sie Clopidogrel in Form des Hydrogensulfats in einer Menge von 2 % (Gew./Vol.) enthält.

6. Wäßrige, injizierbare Zubereitung nach irgendeinem der Ansprüche 1 bis 5, **dadurch gekennzeichnet, daß** das basische Mittel Dinatriumphosphat ist.

7. Wäßrige, injizierbare Zubereitung nach Anspruch 6, **dadurch gekennzeichnet, daß** Dinatriumphosphat in einer annähernd stöchiometrischen Menge, bezogen auf den Wirkstoff, vorhanden ist.

8. Wäßrige, injizierbare Zubereitung nach Anspruch 7, **dadurch gekennzeichnet, daß** sie 0,01 bis 2 % (Gew.-/Vol.) Pluronic F68® enthält.

9. Wäßrige, injizierbare Zubereitung nach Anspruch 8, **dadurch gekennzeichnet. daß** sie 0,5 % (Gew./Vol.) Pluronic F68® enthält.

10. Wäßrige, injizierbare Zubereitung nach Anspruch 9, **dadurch gekennzeichnet, daß** sie 5 bis 15 % (Gew./Vol.) Solutol HS15® enthält.

11. Zubereitung nach Anspruch 10, **dadurch gekennzeichnet, daß** sie 9 % (Gew.-/Vol.) Solutol HS15® enthält.

12. Zubereitung nach irgendeinem der Ansprüche 1 bis 11, **dadurch gekennzeichnet, daß** sie aus einer gefriergetrockneten Formulierung gebildet ist, die 80 % (Gew./Gew.) Clopidogrel in Form des Hydrogensulfats und 20 % (Gew./Gew.) Pluronic F68® enthält, wobei das gefriergetrocknete Produkt in einem Wiederaufnahmelösungsmittel wiederhergestellt eine wäßrige Lösung ergibt, die Dinatriumphosphat in stöchiometrischer Menge, bezogen auf den Wirkstoff, und 9 % (Gew./Vol.) Solutol HS15® enthält.

13. Gefriergetrocknete Formulierung, **dadurch gekennzeichnet, daß** sie als Wirkstoff ein pharmazeutisch annehmbares Salz von Clopidogrel oder Ticlopidin und Pluronic F68® enthält.

14. Gefriergetrocknete Formulierung nach Anspruch 13, dadurch gekennzelchnet, daß der Wirkstoff Clopidogrel in Form des Hydrogensulfats ist.

15. Gefriergetrocknete Formulierung nach Anspruch 14, **dadurch gekennzeichnet, daß** sie 80 % (Gew./Gew.) Clopidogrel in Form des Hydrogensulfats und 20 % (Gew./Gew.) Pluronic F68® enthält.

16. Pharmazeutischer Kit, **dadurch gekennzeichnet, daß** er in Form von zwei sterilen Behältern vorliegt, von denen einer eine gefriergetrocknete Formulierung nach irgendeinem der Ansprüche 13 bis 15 und der andere ein wäßriges Wiederaufnahmemedium, das ein mit der Verabreichung durch Injektion verträgliches basisches Mittel zur Modifizierung des pH-Werts und Solutol HS15® umfaßt, enthalten.

17. Pharmazeutischer Kit, **dadurch gekennzeichnet, daß** er in Form einer Doppelbehälterspritze vorliegt, von denen ein Behälter eine gefriergetrocknete Formulierung nach einem der Ansprüche 13 bis 15 und der andere Behälter ein wäßriges Wiederaufnahmemedium, welches ein mit der Verabreichung durch Injektion verträgliches basisches Mittel zur Modifizierung des pH-Werts und Solutoll HS15® umfaßt, enthalten.

18. Pharmazeutischer Kit nach einem der Ansprüche 16 bis 17, **dadurch gekennzeichnet, daß** das Wiederaufnahmelösungsmittel Dinatriumphosphat als basisches Mittel enthält.

19. Pharmazeutischer Kit nach Anspruch 18, **dadurch gekennzeichnet, daß** das Wiederaufnahmemedium Dinatriumphosphat in einer annähernd stöchiometrischen Menge, bezogen auf den Wirkstoff, enthält.

20. Pharmazeutischer Kit nach Anspruch 19, **dadurch gekennzeichnet, daß** das Wiederaufnahmemedium 5 bis 15 % (Gew./Vol.) Solutol HS15® umfaßt.

21. Pharmazeutischer Kit nach Anspruch 20, **dadurch gekennzeichnet, daß** das Wiederaufnahmemedium 9 % (Gew./Vol.) Solutol HS15® umfaßt.
